**Europäisches Patentamt**

**European Patent Office**

**Office européen des brevets**

(19)

(11) Veröffentlichungsnummer : **0 011 258**
**B1**

(12) **EUROPÄISCHE PATENTSCHRIFT**

(45) Veröffentlichungstag der Patentschrift :
26.01.83

(21) Anmeldenummer : **79104428.2**

(22) Anmeldetag : **09.11.79**

(51) Int. Cl.³ : **A 61 B 17/18, A 61 F 5/04**

(54) **Vorrichtung zur äusseren Festlegung der Teile eines gebrochenen Knochens.**

(30) Priorität : **10.11.78 IT 8494978**
**05.04.79 IT 8493079**

(43) Veröffentlichungstag der Anmeldung :
**28.05.80 Patentblatt 80/11**

(45) Bekanntmachung des Hinweises auf die Patenterteilung : **26.01.83 Patentblatt 83/04**

(84) Benannte Vertragsstaaten :
**AT BE CH DE FR GB LU NL SE**

(56) Entgegenhaltungen :
**CH A 599 781**
**DE A 2 553 782**
**DE A 2 718 515**
**DE A 2 807 364**
**DE B 1 087 318**
**FR A 1 239 266**
**US A 2 238 869**
**US A 2 346 346**
**US A 2 388 482**
**US A 2 393 694**
**US A 3 842 825**
**US A 3 915 162**
**US A 4 096 857**

(73) Patentinhaber : **ORTHOFIX S.r.l.**
**9, Via Tito Speri**
**I-37121 Verona (IT)**

(72) Erfinder : **de Bastiani, Giovanni**
**8, Lungadige Campagnola**
**I-37126 Verona (IT)**
Erfinder : **Brivio, Lodovico Renzi**
**31 Via Zanardelli**
**I-25014 Castenedolo (IT)**
Erfinder : **Aldegheri, Roberto**
**3, Via Cavour**
**I-37057 San Giovanni Lupatoto (IT)**
Erfinder : **Danieletto, Gulseppina**
**24, Via Isonzo**
**I-37126 Verona (IT)**
Erfinder : **Faccioli, Giovanni**
**24/bis Via Dall'Ora G.**
**I-46040 Monzambano (IT)**
Erfinder : **Cavazzana, Andrea**
**24 Via Isonzo**
**I-37126 Verona (IT)**

(74) Vertreter : **Westphal, Klaus, Dipl.-Ing. et al**
**Patentanwälte Dipl.-Ing. Klaus Westphal Dr.rer.nat.**
**Bernd Mussgnug Dr.rer.nat. Otto Buchner Floss-**
**mannstrasse 30a**
**D-8000 München 60 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Vorrichtung zur äusseren Festlegung der Teile eines gebrochenen Knochens

Die Erfindung betrifft eine Vorrichtung zur äußeren Festlegung der Teile eines gebrochenen Knochens, mit einem langgestreckten Mittelkörper, wobei der Mittelkörper zwei in Längsrichtung desselben einstellbare Klemmeinrichtungen für in einem Knochenteil einsetzbare Nägel trägt, wobei die freien Enden des Mittelkörpers jeweils über ein Kugelgelenk mit den Klemmeinrichtungen verbunden sind und wobei die Lage der Klemmeinrichtungen bezüglich des Mittelkörpers durch Feststelleinrichtungen fixierbar ist, und mit einer am Mittelkörper angreifenden Druck- und Zugeinrichtung, welche einen Schraubenbolzen aufweist, wobei die Druck- und Zugeinrichtung abnehmbar mit dem Mittelkörper verbunden ist.

Eine derartige Vorrichtung ist aus der DE-A1-27 18 515 bekannt. Diese bekannte Vorrichtung hat einen verhältnismäßig großen seitlichen Platzbedarf, da auf einem aus einer durchgehenden Stange bestehenden Mittelkörper nach beiden Seiten senkrecht von der Stange abstehende, längs der Strange verschiebbare Teile vorgesehen sind, die an einem Ende von der abnehmbaren Druck- und Zugeinrichtung erfaßt werden können, während das zu dem gebrochenen Glied hinweisende Ende ein Kugelgelenk trägt, das seinerseits mit der Klemmeinrichtung für die Nägel verbunden ist. Selbst nach dem Abnehmen der Druck- und Zugeinrichtung bleibt der seitliche Platzbedarf der bekannten Vorrichtung im wesentlichen gleich groß, da die Druck- und Zugeinrichtung seitlich nicht weiter von der Stange absteht, als die für das Erfassen durch diese Einrichtung vorgesehenen Teile. Außerdem wird bei längerer Benutzung der an einem gebrochenen Knochen angebrachten bekannten Vorrichtung durch die wiederholten Muskelkontraktionen des zugehörigen Gliedes eine Lockerung dieser Vorrichtung bewirkt, die sich auf die feste Halterung des Knochens und somit auf die Heilung nachteilig auswirkt. Die auf der Strange verschiebbaren Teile müssen in Längsrichtung und in Drehrichtung um die Stange festgelegt werden, wobei in mehreren Richtungen starke Drehmomente auftreten. Auch auf die seitlich von der Stange angeordneten Kugelgelenke werden in mehreren Richtungen stärkere Drehmomente ausgeübt, so daß die Stabilität der Anordnung bei längerer Benutzung beeinträchtigt wird. Auch durch das zwischen den Knochen und den in diesen eingesetzten Nägeln ist bei der bekannten Anordnung ein gewisses Spiel nicht zu vermeiden, was die Stabilität weiter herabsetzt.

Durch die Erfindung soll eine Vorrichtung dieser Art so verbessert werden, daß eine auch bei längerer Benutzung absolut sichere Festlegung der Vorrichtung an dem zu schienenden Glied bei einfacher Anbringung und geringem Platzbedarf der Vorrichtung gewährleistet ist.

Diese Aufgabe wird erfindungsgemäß dadurch gelöst, daß der Mittelkörper in an sich bekannter Weise aus zwei parallel zu seiner Längsachse gegeneinander verschiebbaren Teilen besteht, die teleskopartig ineinandergefügt und gegenseitig fixierbar sind, daß die Drehzentren der beiden Kugelgelenke auf der Längsachse des Mittelkörpers liegen, daß jeweils ein Aufnahmesockel für eine Kugel an jedem Ende des Mittelkörpers befestigt ist und ein an der Kugel anliegender Gewindering auf diesen Sockel aufschraubbar ist, daß die Druck- und Zugeinrichtung zwei Hohlzylinder aufweist, die etwa senkrecht abstehende Bolzen tragen, welche in Hohlräume einfügbar sind, die sich an den freien Enden der Teile des Mittelkörpers befinden, wobei der Kopf des Schraubenbolzens auf dem ersten Holzylinder aufliegt und dessen Schaft den ersten Hohlzylinder durchsetzt, fest mit einer Zwischenmuffe zwischen den Hohlzylindern verbunden ist und mit einem Außengewindeabschnitt in ein im zweiten Hohlzylinder vorgesehenes Innengewinde eingreift, und daß die Nägel wenigstens in dem in den Knochen einsetzbaren Abschnitt ein Außengewinde tragen.

Durch die Kombination dieser Merkmale ist eine äußerst einfache, leichte Vorrichtung mit geringem seitlichem Platzbedarf geschaffen, die unmittelbar längs des zu schienenden Gliedes angebracht werden kann und mit Ausnahme der Nägel seitlich nur geringfügig über den Umfang des Mittelkörpers hinausgeht. Die axiale Anordnung von Mittelkörper und Kugelgelenken und die sehr haltungssichere Ausbildung der Kugelgelenke selbst gewährleistet eine außerordentlich gute Stabilität, wobei nur geringe Drehmomente an den Kugelgelenken auftreten. Auch die erfindungsgemäße Ausbildung der Druck- und Zugeinrichtung zum Verspannen der Vorrichtung trägt dazu bei, daß nur in Richtung der Längsachse des Mittelkörpers Kräfte auf die Teile der Vorrichtung ausgeübt werden und daß insbesondere nach Abnehmen der Druck- und Zugeinrichtung keine seitlich aus dem Mittelkörper vorstehenden Teile an diesem vorhanden sind. Darüber hinaus wird durch erfindungsgemäße Anbringung eines Außengewindes auf den Nägeln von vornherein ein lockerer Sitz der Nägel im Knochen und damit der Anlaß für ein Lockern der ganzen Vorrichtung bei Benutzung vermieden. Vor allem wird durch diese Gewinde beim Einsetzen der Nägel in den Knochen die Entstehung von Mikrofrakturen des Knochens vermieden.

Aus der US-A-2 346 346 ist es an sich bereits bekannt, den Mittelkörper, der über feststellbare Kugelgelenke mit den Klemmeinrichtungen verbunden ist, aus zwei teleskopartig ineinandergefügten und gegenseitig fixierbaren Teilen zu bilden. Auch weisen bei dieser Vorrichtung die Kugelgelenke Aufnahmesockel für die Kugel auf, wobei ein Gewindeteil auf den Sockel aufschraubbar ist. Allerdings soll durch diese Gewindeverbindung gleichzeitig sowohl die Kugel im Sockel als auch der Sockel auf dem Mittelteil in Längs- und Drehrichtung festgelegt werden. Dadurch ergibt sich eine verhältnismäßig unstabile Verbindung, die bei größeren Belastungen die eingestellte Lage nicht einhalten kann. Vor allem aber stehen auch bei dieser bekannten Vorrichtung die auf dem Mittelteil längsverschiebbaren Teile, die auch die Kugelgelenke tragen, nach beiden Seiten senkrecht vom Mittelkörper ab, so daß die Vorrichtung die gleichen Nachteile besitzt, wie sie oben mit Bezug auf die Vorrichtung nach der DE-A1-27 18 515 geschildert wurden.

In den Unteransprüchen sind vorteilhafte Ausgestaltungen der erfindungsgemäßen Vorrichtung unter Schutz gestellt. Anhand der Figuren werden Ausführungsbeispiele der Erfindung näher erläutert. Es zeigen :

Figur 1 eine schematische Vorderansicht der erfindungsgemäßen Vorrichtung mit teilweise unterbrochenen Nägeln, die an einem teilweise unterbrochenen Knochen angebracht sind, der teilweise in Ansicht und teilweise im Schnitt dargestellt ist ;

Figur 2 einen senkrechten Mittelschnitt der Vorrichtung gemäß Fig. 1, gedreht um 180° und mit Klemmeinrichtungen, die um 90° gegenüber der Achse der Vorrichtung gedreht sind ;

Figur 3 einen vergrößerten Schnitt eines Kugelgelenks einer Klemmeinrichtung ;

Figur 4 einen zur Längsachse der Vorrichtung senkrechten Schnitt nach der Linie 4-4 in Fig. 2 ;

Figur 5 eine schematische Ansicht eines konisch ausgebildeten Nagels, dessen Gewinde in Fig. 6 teilweise dargestellt ist ;

Figur 7 und 8 zwei Ausführungsvarianten des erfindungsgemäßen, konisch ausgebildeten Nagels ;

Figur 9 eine schematische Ansicht eines konisch ausgebildeten Nagels, der in einem im Schnitt dargestellten geeigneten Tragteil gelagert ist ;

Figur 10 einen Schnitt eines Kugelgelenks einer durch Exzenterbolzen festlegbaren Klemmeinrichtung ;

Figur 11 eine Draufsicht auf den Endteil des zylindrischen Teils des Mittelkörpers ;

Figur 12 eine Untersicht einer Schnellsteckmuffe zum Festlegen des Kugelgelenks gegenüber dem Mittelkörper der Vorrichtung ;

Figur 13, 14 und 15 drei Ausführungsformen einer Feststelleinrichtung des Exzenterbolzens ;

Figur 16 eine teilweise unterbrochene und teilweise geschnittene Ansicht eines Nagels, der zur Aufnahme einer abnehmbaren Spitze in Form eines Bohrers geeignet ist ;

Figur 17 eine teilweise Ansicht des mit einem Gewinde versehenen Teils eines Nagels ;

Figur 18 eine Ansicht einer abnehmbaren Spitze in Form eines Schneckenbohrers ; und

Figur 19, 20 und 21 Teilansichten von drei Nägeln, die in verschieden geformten Spitzen enden.

Die Figuren sind in unterschiedlichem Maßstab gehalten. Gleichen Bezugszahlen entsprechen gleiche oder gleichwertige Teile. Die erfindungsgemäße Vorrichtung weist einen Mittelkörper auf, der aus einem äußeren Zylinder 10 besteht, in den teleskopartig eine Stange 11 eingefügt ist, mit einem Kopf 12, dessen äußere Form der des Zylinders 10 entspricht, in dem das Aufnahmelager eines Bolzens 13 der Druck- und Zugeinrichtung 14 vorgesehen ist.

Der obere Teil 15 des Kopfes 12 ist in irgendeiner bekannten Weise mit einem Sockel 16 fest verbunden, der einen kugelkalottenförmigen Aufnahmesitz für eine Kugel 18 trägt, die über einen Bund 19 und eine Stange 20 fest mit einem festen Backen 21 einer die Nägel tragenden Klemmeinrichtung 17 verbunden ist. Ein Gewindering 22, der auf den Sockel 16 aufschraubbar ist, verankert die Kugel 18 an dem Sockel 16, wobei jedoch vor dem Festziehen des Gewinderings 22 Bewegungen der Klemmeinrichtung 17 gegenüber der Längsachse der Vorrichtung möglich sind. Schraubt man den Gewindering 22 ganz auf den Sockel 16 mittels eines Hankenschlüssels auf, dessen Vorsprünge in die Öffnungen 23 eingeführt werden können, so kann die Kugel 18 gegen den Sockel 16 festgelegt werden ; zur größeren Sicherheit der Festlegung sind in dem Gewindering 22 Bolzenschrauben 24 vorgesehen, die beispielsweise durch Sechskant-Schlüssel anziehbar sind.

Sobald die Schäfte 25 der Nägel in Aufnahmesitze 26, die in den Backen der Klemmeinrichtung 17 vorgesehen sind, eingeführt sind, wird ein beweglicher Backen 27 auf den festen Backen 21 mittels Schrauben 28 angezogen.

In der Basis des Zylinders 10 ist ähnlich der Einrichtung auf dem Kopf 12 der Aufnahmesitz eines Bolzens 13' der Einrichtung 14 vorgesehen, während der untere Teil 15' des Zylinders 10 zur Verankerung und zur Festlegung eines Gelenks und einer Klemmeinrichtung, die mit den vorbeschriebenen identisch sind, mit einem Sockel 16' fest verbunden ist.

Unter Bezugnahme auf Fig. 5 ist zu bemerken, daß der Nagel einen Schaft 25 und einen konischen Kern 29 mit konischem Gewinde aufweist, wie in Fig. 6 näher zu sehen ist, mit einem spiralenförmig verlaufenden Gewindeprofil, dessen Konizität der des Kerns 29 entspricht und das konstante Steigung besitzt, so daß der Vorsprung des Gewindes gegenüber dem Kern, die Neigung der Wände des Gewindes und die Breite der Rillen konstant sind. Ein konisch ausgebildeter Nagel mit den vorbeschriebenen Eigenschaften hat die besondere Eigenschaft, das Spiel beseitigen zu können, das sich unter Umständen zwischen dem Knochen und dem Nagel infolge einer Erweiterung des Aufnahmehohlraums für den Nagel ergibt, die auf ein Nachgeben der Wände des Hohlraums auch in Folge des Zugs oder des Drucks zurückzuführen ist, dem die Vorrichtung ausgesetzt ist, und zwar durch einfaches weiteres Einschrauben des Nagels, das offensichtlich so weit erfolgen muß, als es zur Beseitigung des entstandenen Spiels nötig ist.

Es ist klar, daß nach dieser Ausführung sowohl der Kern des Nagels als auch das Gewinde durch ihre Konizität beide zur Beseitigung des Spiels beitragen.

Unter Bezugnahme auf Fig. 7 ist festzustellen, daß der Kern 30 des dargestellten Nagels einen konischen Verlauf hat, während das Gewinde 31 in Form einer zylindrischen Schraube verläuft, weshalb an der Beseitigung des Spiels vorwiegend der Kern des Nagels beteiligt ist. Bei der Ausführungsvariante der Fig. 8 ist das Gegenteil der Fall, weil der Kern 32 zylindrisch ist, während das Gewinde 33 als konische Spirale verläuft, weshalb in diesem Fall die Beseitigung des Spiels überwiegend vom Gewinde abhängt.

Es ist klar, daß die drei Arten von Nägeln mit einer einzigen Klemmeinrichtung verwendet werden können, auch wenn die von ihnen entfaltete Funktion von Nagel zu Nagel verschieden ist. Anders als in Fig. 5 dargestellt, kann der Nagel einen Kern und ein Gewinde gemäß der Beschreibung zu den Figuren 6, 7 und 8 nur in den in die Rindenbereiche des Knochens eindringenden Zonen aufweisen, wodurch die Erfindung in den wesentlichen Merkmalen nicht verändert wird.

Außerdem ist klar, daß, ohne daß dies in den beigefügten zeichnungen dargestellt ist, ein erfindungsgemäßer Nagel teilweise einen an sich bekannten, zylindrischen Kern mit einem Gewinde in Form einer zylindrischen Schraube aufweisen kann, der sich in einen Kern und/oder in ein Gewinde mit konischem Verlauf fortsetzt.

Praktische Versuche mit erfindungsgemäßen Schrauben mit Knochen durchschnittlicher kortikaler Kompaktheit haben zu ausgezeichneten Ergebnissen geführt bei Schrauben mit einem Schaft von 6 mm $\varnothing$, mit einem Kern und/oder Gewinde mit einer Konizität von 1 : 50, mit einem Gewinde von 1,75 mm Steigung, mit einer Neigung der Wände des Gewindes von 60°, mit einer Mindestbreite der Rille von 0,69 mm und einer Gewindetiefe von 0,75 mm, wie in Fig. 6 gezeigt, ohne daß dadurch die Erfindung auf diese Abmessungen festgelegt werden soll.

Unter Bezugnahme insbesondere auf Fig. 9 ist zu bemerken, daß die entsprechend der obigen Beschreibung hergestellten Nägel einen Schaft oder eine Stange 34 aufweisen können, die in den Hohlraum eines Tragteils 35 einschraubbar ist, der zwischen die Backen der Klemmeinrichtungen 17 eingeführt werden kann, so daß der Nagel durch einen Hakenschlüssel gedreht werden kann, dessen Haken in Hakenlöcher 36 eingreifen können, um dadurch das vorbeschriebene Spiel zu beseitigen, ohne die Backen der Klemmeinrichtungen 17 lösen zu müssen.

Um den Zustand der Anlage während der Beseitigung des erwähnten Spiels nicht im geringsten zu stören, ist es unerläßlich, daß die Steigung des Gewindes am Schaft genau der Steigung des Gewindes am Kern des Nagels entspricht.

Sobald die Nägel im Knochen oberhalb und unterhalb der Bruchlinie in Ebenen befestigt sind, die auch von der Sagittalebene oder einer durch die Längsachse des Knochens gehenden Ebene abweichen können, bringt man den Mittelkörper der Vorrichtung in eine Lage, in der seine Längsachse parallel zur Längsachse des Knochens verläuft, und führt die Schäfte der Nägel oder ihre Tragteile in die Aufnahmesitze 26 der Backen der Klemmeinrichtungen 17 ein, um anschließend die endgültige Festlegung der Klemmeinrichtungen gegenüber dem Mittelkörper und der Backen der Klemmeinrichtungen gegenüber den Nägeln vorzunehmen.

Auf diese Weise ist die Vorrichtung nunmehr in der Lage, Druck oder Zug auf den Knochen durch den Einsatz der Einrichtung 14 auszuüben, zu der ein Hohlzylinder 37, an dessen unterem Ende der Bolzen 13' befestigt ist, ein zweiter Hohlzylinder 38, an dem der Bolzen 13 befestigt ist, eine Schraube 39, deren Kopf 40 im Hohlzylinder 38 gelagert ist, und eine Muffe 41 gehören, die fest mit einem Stangenabschnitt 42 der Schraube 39 verbunden ist, deren Außengewinde in das Innengewinde des Hohlzylinders 37 eingreift. Sobald die Bolzen 13, 13' in die geeigneten, jeweils in den Teilen 12 und 10 vorgesehenen Hohlräume eingeführt sind, ist es ausreichend, die Schraube 39 durch Betätigung des Kopfes 40 einzuschrauben oder aufzuschrauben, um je nach Bedarf die gegenseitige Annäherung oder Entfernung der Teile 10 und 12 des Mittelkörpers der Festlegungsvorrichtung mit axialen Verschiebungen der Stange 11 im Inneren von 10 zu bewirken.

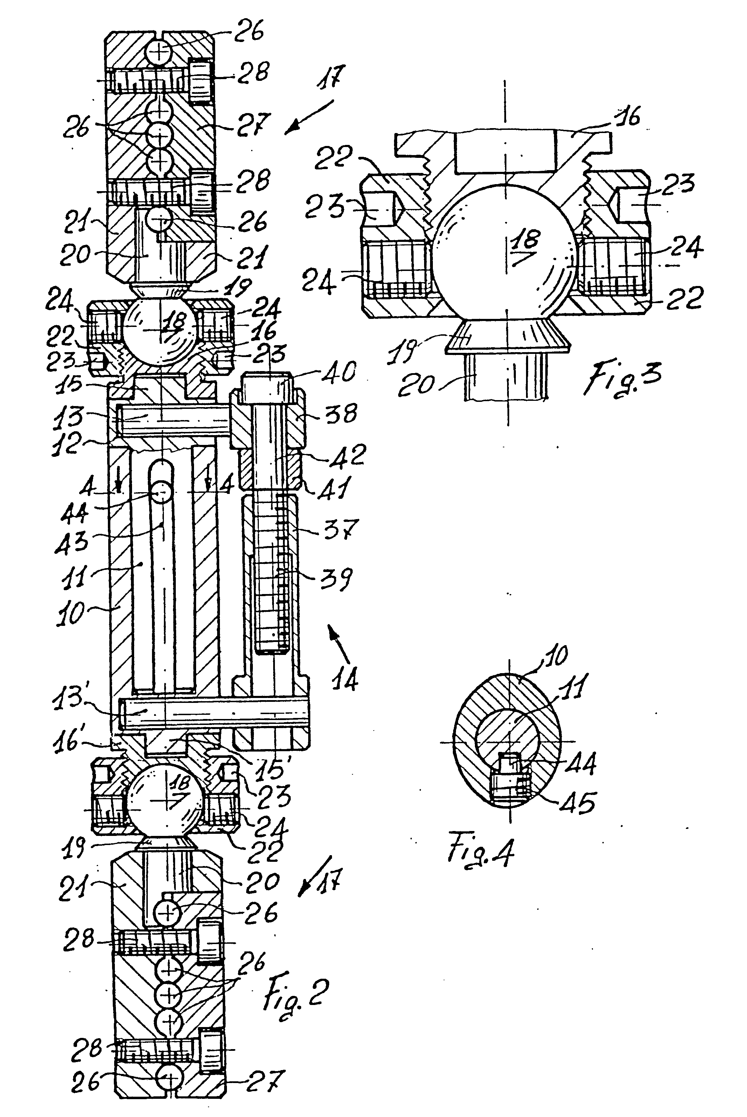

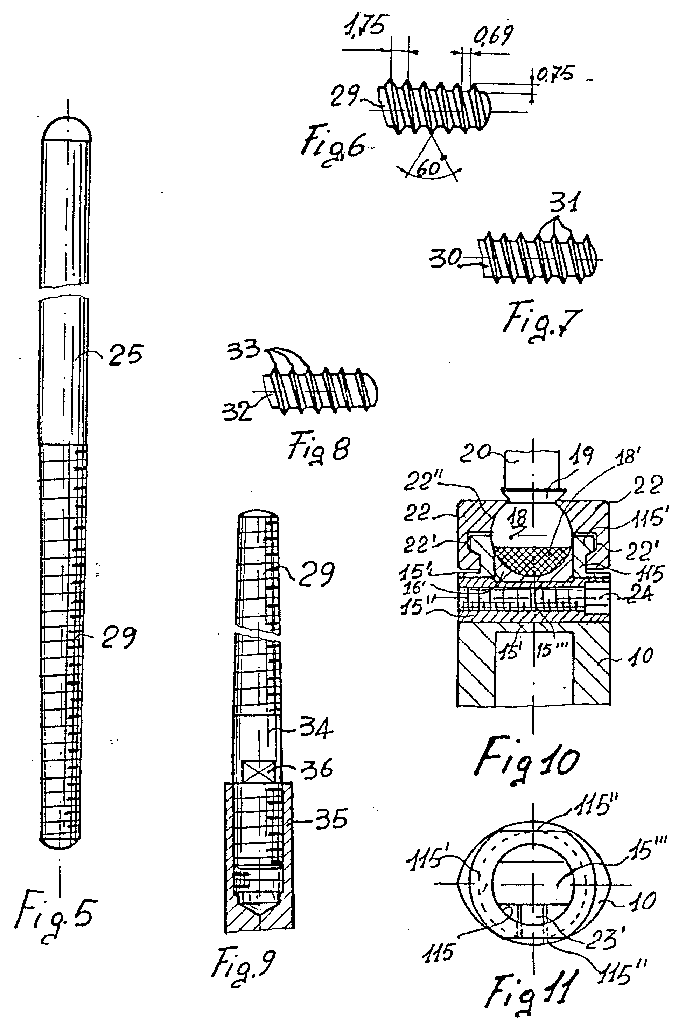

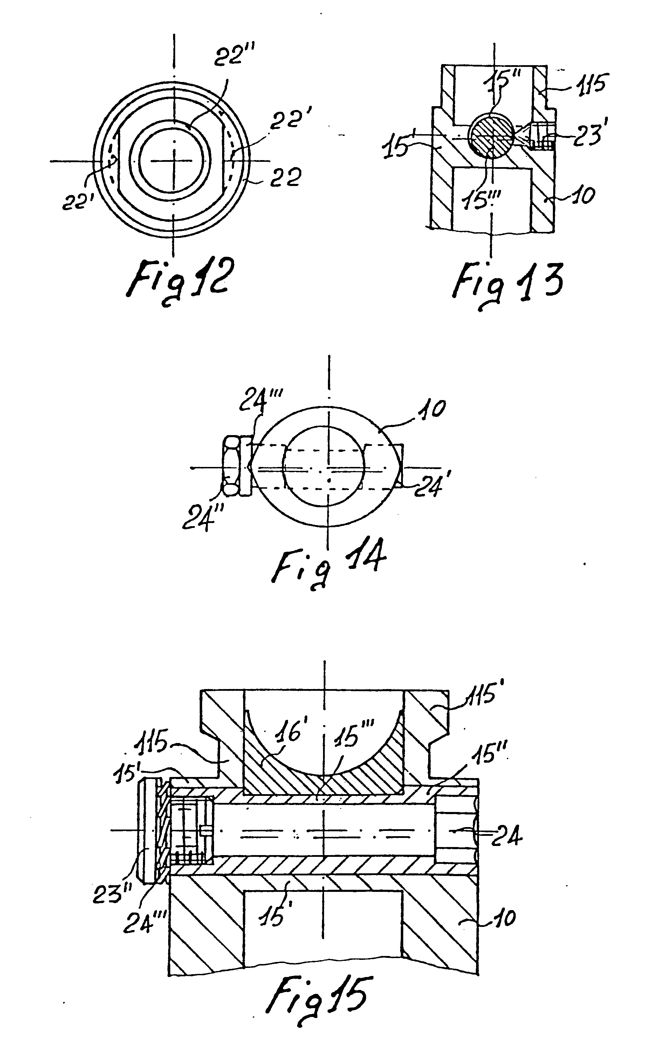

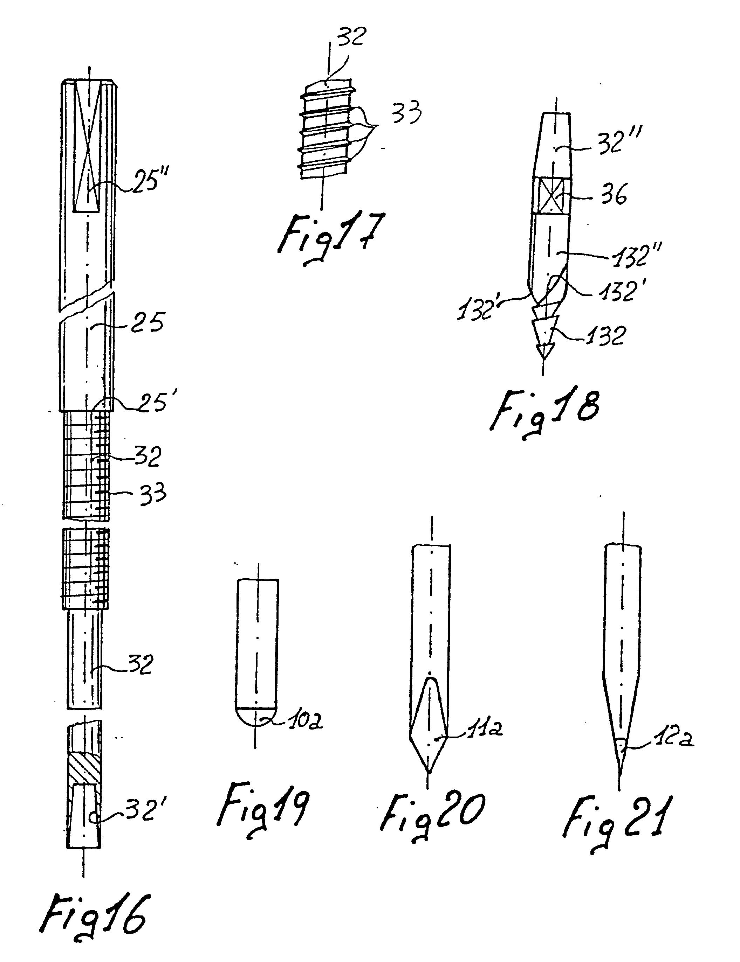

Die Stange 11, siehe dazu Fig. 2, ist mit einer Nut 43 versehen, in der ein Anschlag- und Festlegungspflock 44 vorsteht. Dieser Pflock 44 ist, siehe dazu Fig. 4, mit einer Schraube 45 fest verbunden, deren Außengewinde in das Innengewinde eingreift, das an geeigneter Stelle im Teil 10 vorgesehen ist.

Sobald der gewünschte Druck oder Zug, der auch durch eine Meßskala überprüfbar ist, die nicht dargestellt ist, jedoch auf der Stange 11 angebracht werden kann, erreicht ist, wird die Schraube 45 fest angezogen und der Pflock 44 dazu gebracht, gegen den Boden der Nut 43 zu drücken und durch Reibung die Teile 10 und 12 des Mittelkörpers der Vorrichtung gegeneinander festzulegen. Die Einrichtung 14 kann daher von der Vorrichtung entfernt werden, indem sie entspannt und sodann die Bolzen 13-13' aus den entsprechenden Aufnahmesitzen herausgezogen werden.

Es ist klar, daß die zuletzt beschriebene Festlegungseinrichtung durch Durchgangsbohrungen auf der Stange 11 und auf dem Zylinder 10 ersetzt werden kann, in die Anschlag- und Befestigungsbolzen eingeführt werden können, ohne daß dadurch der Erfindungsbereich verlassen wird.

Gemäß Fig. 10 bis 12 ist in dem Ende 15' des Zylinders 10 des Mittelkörpers ein zylindrischer Stift 15" gelagert, dessen Mittelteil 15''' gegenüber seinen Enden exzentrisch ist.

Das Ende 15' setzt sich in einen Hohlzylinder 115 mit einem ringförmigen Vorsprung 115' fort, der bei 115" längs zweier paralleler und die äußere Oberfläche des Hohlzylinders 115 berührender Ebenen abgeschnitten ist, wobei die abgeschnittenen Stellen 115" das Einführen und die nachfolgende Drehbewegung einer Schnellsteckmuffe 22 mit ringförmigem Hohlraum 22' gestatten, der formschlüssig zum ringförmigen Vorsprung 115' ausgebildet ist.

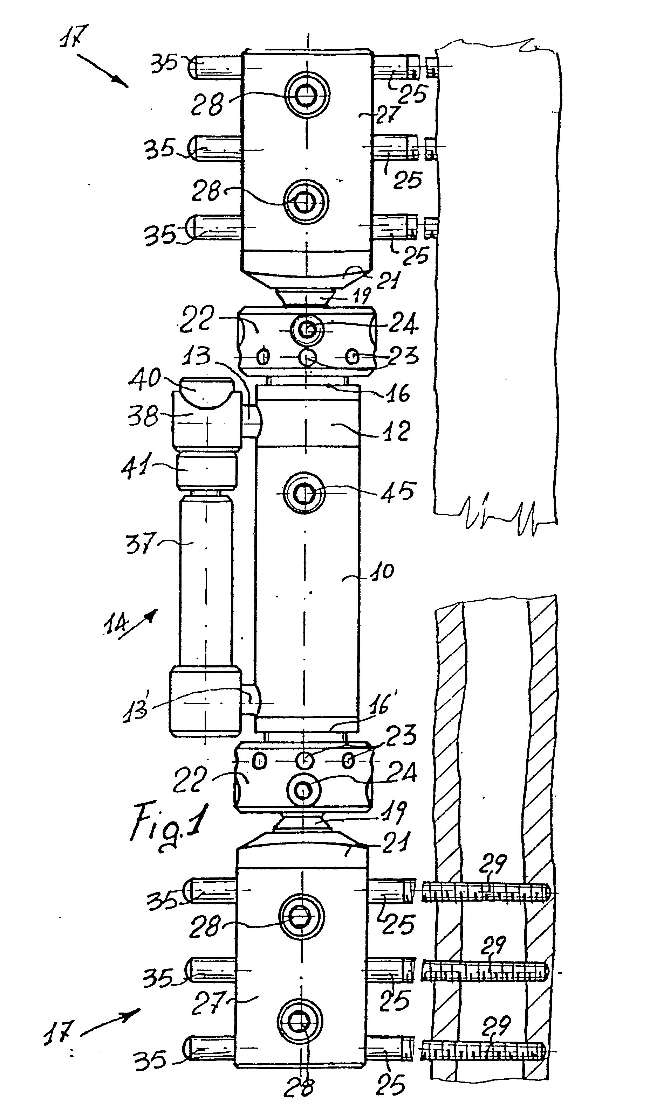

In dem Hohlzylinder 115 ist eine Schale 16' gelagert mit einem kugelkalottenförmigen Aufnahmesitz für die Kugel 18, die über den Bund 19 und die Stange 20 fest mit der festen Backe 21 einer nicht dargestellten, die Nägel tragenden Klemmeinrichtung entsprechend der in Fig. 1 gezeigten Klemmeinrichtung 17 verbunden ist.

Die genannte Schale 16' liegt mit ihrem unteren Teil auf dem exzentrischen Teil 15''' des Bolzens 15'' auf, weshalb nach dem Aufstecken der Muffe 22 auf den Hohlzylinder 115 und nach Drehung derselben um eine Vierteldrehung, um den ringförmigen Vorsprung 115' und den Hohlraum 22' zum Eingriff zu bringen, die Kugel 18 zwischen der Schale 16' und einen Ring 22'' mit kugelkalottenförmiger Oberfläche festgehalten wird.

Die Kugel 18, die zunächst so festgehalten ist, daß sie noch eine Drehbewegung ausführen kann, wird in einer beliebigen Stellung festgelegt, indem der Bolzen 15'' gedreht wird, so daß er mit seinem exzentrischen Teil 15'' die Schale 16' gegen die Kugel 18 und diese gegen den Ring 22'' drückt.

Um die Sicherheit der Festlegung der Kugel 18 zu erhöhen, kann ihre äußere Oberfläche ganz oder teilweise mit einer Rändelung 18' versehen sein ; gleichermaßen können auch die Oberflächen, die mit der Kugel 18 in Berührung kommen, mit einer Rändelung versehen sein.

An einem Ende des Bolzens 15'' befindet sich eine Sechskantschraube 24, um dem Bolzen mittels eines Sechskantschlüssels die erforderlichen Drehbewegungen zu erteilen.

Sobald die Festlegung der Kugel 18 erfolgt ist, wird die Lage des Bolzens 15'' durch eine Klemm-Madenschraube 23' festgelegt, die auf den exzentrischen Teil 15''' gemäß der Darstellung in Fig. 13 wirkt. Diese Festlegung kann unterstützt werden durch die Festlegung des Bolzens 15' durch eine Mutter 24' und eine Gegenmutter 24'', wobei ein Federring 24''' gemäß der Darstellung in Fig. 14 dazwischengelegt wird, oder durch die Zugwirkung einer Schraube 23'' über einen beigelegten Federring 24''' auf den Bolzen 15'', der seitlich nicht herausgezogen werden kann, weil er daran durch die Schale 16' gehindert wird, die mit ihrem unteren Teil gemäß Fig. 15 auf dem Teil 15''' aufliegt.

Bei den Nägeln, die mit zweiseitiger Feststellvorrichtung gemäß Fig. 16 zu verwenden sind, weist der Schaft 25 einen Durchmesser auf, der geringfügig größer ist als der Kern 32 und das Gewinde 33, so daß ein ringförmiger Vorsprung 25' einen Anschlag des Nagels an der Rindenzone des Knochens bildet, und es ist einleuchtend, daß drei vor oder nach einer Bruchfläche in der Weise eingeführte Nägel, daß einer derselben auf der den beiden anderen gegenüberliegenden Seite in den Knochen eindringt, mit ihren Anschlägen 25' gegenseitige Verschiebungen zwischen dem Knochen und der Vorrichtung längs der Achse der Nägel verhindern, und zwar auch dann, wenn sich im Bereich des Kerns 32 und/oder des Gewindes 33 ein Spiel zwischen Knochen und Nagel ergeben sollte.

Ebenfalls in Fig. 16 ist zu erkennen, daß an dem freien Ende des Schaftes 25 der Aufnahmesitz 25'' vorgesehen ist, um den Nagel an ein manuelles oder elektrisches Gerät zur Perforation des Knochens zu befestigen, beispielsweise eine Bohrmaschine, während am anderen Ende der Kern 32 einen Aufnahmekegel 32' für eine als Bohrer ausgebildete Spitze trägt, die abgenommen werden kann, sobald der Nagel im Knochen befestigt ist.

Unter Bezugnahme auf Fig. 18 ist zu erkennen, daß eine solche Spitze einen Werkzeugkegel 32'', der formschlüssig zu dem Aufnahmesitz 32' ausgebildet ist, und Einhakbohrungen 36 trägt, um durch eine Drehbewegung mittels eines Hakenschlüssels in den konischen Aufnahmesitz 32' eingesteckt werden zu können. Diese Spitze besitzt die Form eines Schneckenbohrers und besteht aus einem Stiel, der in einer konischen Schraube 132 endet, die sich in seitliche schraubenlinienförmige Schneiden 132' mit wesentlich größerer Steigung fortsetzt, welche einen offenen Hohlraum 132'' zur Aufnahme der Bohrabfälle begrenzen.

Es ist klar, daß der Hohlraum 132'' so dimensioniert ist, daß er die Bohrabfälle restlos aufnehmen kann, und daß er auch teilweise im Inneren der Spitze selbst vorgesehen sein kann.

Bezugnehmend auf Fig. 19 ist festzustellen, daß ein in eine abgerundete « Spitze » 10a auslaufender Nagel in einen Knochen nur dann eingeführt werden kann, wenn zuvor in demselben die entsprechende Bohrung hergestellt worden ist, während die Nägel gemäß Figuren 20 und 21, die in einer lanzenförmigen Spitze 11a bzw. in einer konischen Spitze 12a enden, auch durch einfaches Einschlagen eingetrieben werden können.

Praktische Versuche mit Nägeln mit fester oder abnehmbarer Spitze, wie die in Fig. 18 dargestellte, haben zu ausgezeichneten Ergebnissen geführt, was die Vermeidung von Mikrofrakturen durch das Einschrauben betrifft, wenn die Gewindetiefe 1 mm beträgt, und haben zu ausgezeichneten Ergebnissen in bezug auf die Verhinderung von Relativbewegungen zwischen Knochen und Festlegungsvorrichtung durch Anschläge geführt, die einen Mindestvorsprung gegenüber dem Gewindeteil von 0,5 mm aufweisen.

**Ansprüche**

1. Vorrichtung zur äußeren Festlegung der Teile eines gebrochenen Knochens, mit einem langgestreckten Mittelkörper (10, 11), wobei der Mittelkörper (10, 11) zwei in Längsrichtung desselben einstellbare Klemmeinrichtungen (17) für in einen Knochenteil einsetzbare Nägel (25, 29-36, 132) trägt, wobei die freien Enden des Mittelkörpers (10, 11) jeweils über ein Kugelgelenk (16, 16', 18, 22) mit den Klemmeinrichtungen (17) verbunden sind und wobei die Lage der Klemmeinrichtungen (17) bezüglich des Mittelkörpers (10, 11) durch Feststelleinrichtungen (22, 24, 16, 16') fixierbar ist, und mit einer am Mittelkörper (10, 11) angreifenden Druck- und Zugeinrichtung (14), welche einen Schraubenbolzen (39) aufweist, wobei die Druck- und Zugeinrichtung (14) abnehmbar mit dem Mittelkörper (10, 11) verbunden ist, dadurch gekennzeichnet, daß der Mittelkörper (10, 11) in an sich bekannter Weise aus zwei parallel zu seiner Längsachse gegeneinander verschiebbaren Teilen (10, 11) besteht, die teleskopartig ineinandergefügt und gegenseitig fixierbar sind, daß die Drehzentren der beiden Kugelgelenke (16, 16', 18, 22) auf der Längsachse des Mittelkörpers (10, 11) liegen, daß jeweils ein Aufnahmesockel (16, 16') für eine Kugel (18) an jedem Ende des Mittelkörpers (10, 11) befestigt ist und ein an der Kugel (18) anliegender Gewindering (22) auf diesen Sockel (16, 16') aufschraubbar ist, daß die Druck- und Zugeinrichtung (14) zwei Hohlzylinder (37, 38) aufweist, die etwa senkrecht abstehende Bolzen (13, 13') tragen, welche in Hohlräume einfügbar sind, die sich an den freien Enden der Teile (10, 11) des Mittelkörpers befinden, wobei der Kopf (40) des Schraubenbolzens (39) auf dem ersten Hohlzylinder (38) aufliegt und dessen Schaft (42) den ersten Hohlzylinder (38) durchsetzt, fest mit einer Zwischenmuffe (41) zwischen den Hohlzylindern (37, 38) verbunden ist und mit einem Außengewindeabschnitt (39) in ein im zweiten Hohlzylinder (37) vorgesehenes Innengewinde eingreift, und daß die Nägel (25, 29-36, 132) wenigstens in dem in den Knochen einsetzbaren Abschnitt (29, 30, 32) ein Außengewinde (31, 33) tragen.

2. Vorrichtung nach Anspruch 1, dadurch gekennzeichnet, daß die Nägel einen zylindrischen Schaft (25) und einen konischen Kern (29) aufweisen, der mit einem spiralförmigen Außengewinde mit konstanter Steigung versehen ist, wobei die äußeren Konizitäten des Kerns (29) und des Außengewindes parallel sind und wobei der Schaft (25) in einem Aufnahmesitz (26) der Backen (21, 27) der Klemmeinrichtungen (17) festlegbar ist.

3. Vorrichtung nach Anspruch 1, dadurch gekennzeichnet, daß die Nägel einen Zylindrischen Schaft (25) und einen konischen Kern (30) aufweisen, der ein zylindrisches Außengewinde (31) mit konstanter Steigung trägt, wobei der Schaft (25) in einem Aufnahmesitz (26) der Backen (21, 27) der Klemmeinrichtungen (17) festlegbar ist.

4. Vorrichtung nach Anspruch 2 oder 3, dadurch gekennzeichnet, daß der zylindrische Schaft (34) ein zylindrisches Außengewinde trägt, dessen Steigung der des Kerngewindes (31, 33) entspricht, und daß der Schaft (34) in einen Hohlraum eines Tragteils (35) eingeschraubt ist, dessen Innengewinde dem Außengewinde des Schaftes entspricht, wobei der Tragteil (35) in einem Aufnahmesitz (26) der Backen (21, 27) der Klemmeinrichtungen (17) festlegbar ist.

5. Vorrichtung nach einem der vorangehenden Ansprüche, dadurch gekennzeichnet, daß zur zusätzlichen Feststellung der Kugelgelenke (16, 16', 18, 22) Schrauben (24) vorgesehen sind, die den Gewindering (22) durchsetzen.

6. Vorrichtung nach Anspruch 1, dadurch gekennzeichnet, daß die Feststelleinrichtungen der Kugelgelenke (16, 16', 18, 22) aus einem eine Bohrung des jeweiligen Endes (15, 15') des Mittelkörpers (10, 11) durchsetzenden zylindrischen Bolzen (15") mit exzentrischem Mittelteil (15'''), aus einer kugelkalottenförmigen Schale (16') zur Aufnahme der Kugel (18) des Gelenks, die in einem Hohlzylinderabschnitt (115) am Ende (15') des Mittelkörpers (10, 11) beweglich ist und mit ihrem unteren Teil auf dem exzentrischen Teil (15''') des Bolzens (15") aufliegt, aus einer Schnellsteckmuffe (22), die auf den Hohlzylinderabschnitt (115) aufgesteckt ist und einen Ring (22") mit kugelkalottenförmiger Oberfläche aufweist, und aus Einrichtungen (23', 23", 24', 24", 24''') zum Festlegen des zylindrischen Bolzens (15") in einer stabilen Drehlage bestehen.

7. Vorrichtung nach Anspruch 6, gekennzeichnet durch Rändelungen (18') auf der Kugel (18) und/oder auf den Oberflächen der Schale (16') und der Schnellsteckmuffe (22).

8. Vorrichtung nach Anspruch 6 oder 7, dadurch gekennzeichnet, daß die Einrichtungen zum Festlegen des zylindrischen Bolzens (15") aus einer Madenschraube (23') bestehen, die gegen den exzentrischen Teil (15''') des Bolzens (15") anliegt.

9. Vorrichtung nach Anspruch 6 oder 7, dadurch gekennzeichnet, daß die Einrichtungen zum Festlegen des zylindrischen Bolzens (15") aus Muttern (24', 24") und/oder Schrauben (23') bestehen, die einen axialen Zug auf den Bolzen (15") ausüben, wobei die Zugwirkung durch mindestens einen Federring (24''') abgefedert ist.

10. Vorrichtung nach Anspruch 1, dadurch gekennzeichnet, daß die Nägel einen glatten, in eine Spitze auslaufenden Kern (32) aufweisen, mindestens ein Teil des Kerns (32) mit einem Gewinde (33) versehen ist, das radial über den Kern vorsteht, und mindestens ein Schaft (25) vorgesehen ist, der radial leicht über das Gewinde (33) vorsteht.

11. Vorrichtung nach Anspruch 10, dadurch gekennzeichnet, daß die Nägel eine abnehmbare Spitze aufweisen, die aus einem spitz zulaufenden Kern (132) mit einem konischen Schraubengewinde besteht, das nach rückwärts in einen zylindrischen Abschnitt mit steilen schraubenlinienförmigen Schneiden (132') übergeht, welche einen Hohlraum (132") zur Aufnahme des Bohrabfalls begrenzen.

**Claims**

1. Device for externally immobilizing the portions of a fractured bone, comprising an elongated central body (10, 11), said central body (10, 11) supporting two clamping means (17) adjustable in longitudinal direction for nails (25, 29-36, 132) to be inserted into a portion of a bone, each of the free ends of the central body (10, 11) being connected to a clamping means (17) through a ball-and-socket joint (16, 16', 18, 22) wherein the position of the clamping means (17) with respect to the central body (10, 11) can be locked by locking means (22, 24, 16, 16'), and comprising a compression and traction device (14) acting upon the central body (10, 11) and provided with a screw bolt (39), the compression and traction device (14) being removably connected to the central body (10, 11), characterized in that the central body (10, 11) consists of two parts (10, 11) slidable with respect to each other parallel to the longitudinal axis in a manner known in itself, which are telescoped and can be mutually locked, that the centers of rotation of the two ball-and-socket joints (16, 16', 18, 22) are lying on the longitudinal axis of the central body (10, 11), that one receptacle (16, 16') is attached on each end of the central body (10, 11) for each ball (18), a ring nut (22) bearing against the ball (18) being screwable on said receptacle (16, 16'), that the compression and traction device (14) is provided with two hollow cylinders (37, 38) having bolts (13, 13') projecting essentially at right angles which can be engaged into cavities provided at the free ends of parts (10, 11) of the central body, the head (40) of the screw bolt (39) bearing against the first hollow cylinder (38) while its shaft (42) traverses the first hollow cylinder (38), is tightly connected with an intermediate bushing (41) between the hollow cylinders (37, 38) and engages an internal thread provided in the second hollow cylinder (37) with an externally threaded section (39), and that the nails (25, 29-36, 132) are provided with an external thread (31, 33) at least in their section to be inserted into the bone.

2. Device according to claim 1, characterized in that the nails are provided with a cylindrical shaft (25) and with a conical core (29) having a helical external thread with constant pitch, the external tapers of the core (29) and of the external thread being parallel and the shaft (25) being arrestable in a receptacle (26) of the jaws (21, 27) of the clamping means (17).

3. Device according to claim 1, characterized in that the nails are provided with a cylindrical shaft (25) and with a conical core (30) having a cylindrical external thread (31) with constant pitch, the shaft (25) being arrestable in a receptacle (26) of the jaws (21, 27) of the clamping means (17).

4. Device according to claim 2 or 3, characterized in that the cylindrical shaft (34) has a cylindrical external thread the pitch of which corresponds to that of the thread of the core (31, 33), and that the shaft (34) is screwed into a cavity of a supporting means (35) the internal thread of which corresponds to the external thread of the shaft, the supporting means (35) being arrestable in a receptacle (26) of the jaws (21, 27) of the clamping means (17).

5. Device according to one of the preceding claims, characterized in that screws (24) traversing the ring nut (22) are provided for additional locking of ball-and-socket joints (16, 16', 18, 22).

6. Device according to claim 1, characterized in that the locking devices of the ball-and-socket joints (16, 16', 18, 22) are composed of a cylindrical bolt (15") with an eccentric middle section (15''') traversing a bore of the corresponding end (15, 15') of the central body (10, 11), of a spherical calotte (16') for receiving the ball (18) of the joint, which can be moved within a hollow cylinder section (115) at the end (15') of the central body (10, 11) and which rests with its lower portion on the eccentric section (15''') of the bolt (15"), of a quick-fitting bushing (22) which is slipped on the hollow cylinder section (115) and has a ring (22") with a surface in the form of a spherical calotte, and of means (23', 23", 24', 24", 24''') for locking the cylindrical bolt (15") in a stable angular position.

7. Device according to claim 6, characterized by knurlings (18') on the ball (18) and/or the surfaces of the calotte (16') and of the quick-fitting bushing (22).

8. Device according to claim 6 or 7, characterized in that the means for locking the cylindrical bolt (15") consist of a set screw (23') engaging the eccentric section (15''') of the bolt (15").

9. Device according to claim 6 or 7, characterized in that the means for locking the cylindrical bolt (15") consist of nuts (24', 24") and/or screws (23') exerting axial traction upon the bolt (15"), the pull being damped by at least one spring washer (24''').

10. Device according to claim 1, characterized in that the nails have a smooth tapering core (32), that at least one portion of the core (32) is provided with a thread (33) projecting radially over the core, and that at least one shaft (25) slightly projecting over the thread (33) is provided.

11. Device according to claim 10, characterized in that the nails have a removable tip consisting of a tapered core (132) with a conical screw thread verging backwards into a cylindrical section with steep helical cutting edges (132') defining a hollow space (132") for receiving the bore chips.

**Revendications**

1. Dispositif pour l'immobilisation extérieure des parties d'un os fracturé, comportant un corps central (10, 11) allongé, ce corps central (10, 11) portant deux dispositifs de serrage (17) réglables en direction longitudinale pour des clous (25, 29-36, 132) à mettre en place dans une partie d'os, chacune des extrémités libres du corps central (10, 11) étant reliée au dispositif de serrage par un joint à rotule (16, 16', 18, 22), et la position du dispositif de serrage (17) par rapport au corps central (10, 11) pouvant être fixée par des dispositifs de blocage (22, 24, 16, 16'), et comportant un mécanisme de compression et de traction (14) agissant sur le corps central (10, 11) et pourvu d'un boulon fileté (39), le mécanisme de compression et de traction (14) étant attaché au corps central (10, 11) de façon amovible, caractérisé en ce que le corps central (10, 11) est composé, d'une façon connue en soi, de deux parties (10, 11) coulissant l'une par rapport à l'autre parallèlement à l'axe longitudinal, s'emboîtant en forme de télescope, et qui peuvent être arrêtées l'une par rapport à l'autre, que les centres de rotation des deux joints à rotule (16, 16', 18, 22) sont disposés sur l'axe longitudinal du corps central (10, 11), que pour chacune des rotules (18) un socle de logement (16, 16') est fixé à chaque bout du corps central (10, 11) et qu'une bague filetée (22) portant contre la rotule (18) peut être vissée sur ce socle (16, 16'), que le mécanisme de compression et de traction (14) comporte deux cylindres creux (37, 38) qui portent des boulons (13, 13') faisant saillie sensiblement à angle droit, qui peuvent s'insérer dans des cavités prévues aux bouts libres des parties (10, 11) du corps central, la tête (40) du boulon fileté (39) portant contre le premier cylindre creux (38), alors que sa tige (42) traverse le premier cylindre creux (38), est solidaire d'un manchon intermédiaire (41) entre les cylindres creux (37, 38) et s'engage avec une partie filetée (39) dans une partie taraudée dans le deuxième cylindre creux (37), et que les clous (25, 29-36, 132) portent un filet extérieur (31, 33) au moins sur la partie prévue à être insérée dans l'os.

2. Dispositif selon la revendication 1, caractérisé en ce que les clous présentent une tige cylindrique (25) et un noyau conique (29) pourvu d'un filet extérieur hélicoïdal à pas constant, les conicités extérieures du noyau (29) et du filet extérieur étant parallèles et la tige (25) pouvant être arrêtée dans un logement (26) des joues (21, 27) des dispositifs de serrage (17).

3. Dispositif selon la revendication 1, caractérisé en ce que les clous présentent une tige cylindrique (25) et un noyau conique (30) portant un filet extérieur (31) cylindrique à pas constant, la tige (25) pouvant être arrêtée dans un logement (26) des joues (21, 27) des dispositifs de serrage (17).

4. Dispositif selon l'une des revendications 2 ou 3, caractérisé en ce que la tige cylindrique (34) porte un filet extérieur cylindrique dont le pas correspond à celui du filet du noyau (31, 33) et que la tige (34) est vissée dans une cavité d'une attache (35) dont le filet intérieur correspond au filet extérieur de la tige, l'attache (35) pouvant être arrêtée dans un logement (26) des joues (21, 27) des dispositifs de serrage (17).

5. Dispositif selon l'une quelconque des revendications précédentes, caractérisé en ce que des vis (24) traversant la bague filetée (22) sont prévues pour le blocage supplémentaire des joints à rotule (16, 16', 18, 22).

6. Dispositif selon la revendication 1, caractérisé en ce que les dispositifs de blocage des joints à rotule (16, 16', 18, 22) se composent d'un boulon cylindrique (15") avec une partie médiane excentrique (15''') traversant un percement du bout correspondant (15, 15') du corps central (10, 11), d'une coupe en calotte (16') pour recevoir la rotule (18) du joint, qui est mobile dans une partie du cylindre creux (115) au bout (15') du corps central (10, 11) et qui porte avec sa partie inférieure sur la partie excentrique (15''') du boulon (15"), d'un manchon de raccordement rapide (22) rapporté sur la partie de cylindre creux (115) et pourvu d'un anneau (22") à surface en forme de coupe en calotte, et de dispositifs (23', 23", 24', 24", 24''') pour arrêter le boulon cylindrique (15") dans une position angulaire stable.

7. Dispositif selon la revendication 6, caractérisé par des moletages (18') sur la rotule (18) et/ou sur les surfaces des coupes (16') et du manchon de raccordement rapide (22).

8. Dispositif selon l'une des revendications 6 ou 7, caractérisé en ce que les dispositifs pour arrêter le boulon cylindrique (15") consistent en une vis sans tête (23') portant contre la partie excentrique (15''') du boulon (15").

9. Dispositif selon l'une des revendications 6 ou 7, caractérisé en ce que les dispositifs pour arrêter le boulon cylindrique (15") se composent d'écrous (24', 24") et/ou de vis (23') exerçant une traction axiale sur le boulon (15"), l'effet de traction étant amorti par au moins une rondelle ressort (24''').

10. Dispositif selon la revendication 1, caractérisé en ce que les clous présentent un noyau lisse (32) se terminant en une pointe, qu'au moins une partie du noyau (32) est pourvue d'un filet (33) saillant radialement sur le noyau, et qu'au moins une tige (25) saillant légèrement sur le filet (33) est prévue.

11. Dispositif selon la revendication 10, caractérisé en ce que les clous possèdent une pointe amovible consistant en un noyau (132) effilé avec un filetage de vis conique qui se convertit vers l'arrière en une section cylindrique équipée de tranchants hélicoïdaux (132') à pente raide qui définissent un creux (132") pour recevoir la rognure.

**0 011 258**

Fig.1

0 011 258

Fig. 2

Fig. 3

Fig. 4

Fig.5

Fig.6

Fig.7

Fig 8

Fig.9

Fig 10

Fig 11

Fig 12

Fig 13

Fig 14

Fig 15

Fig16

Fig17

Fig18

Fig19

Fig20

Fig21